Europäisches Patentamt

European Patent Office

Office européen des brevets

(11) Numéro de publication: **0 192 900 B1**

# FASCICULE DE BREVET EUROPEEN

(45) Date de publication de fascicule du brevet: **22.04.92**   (51) Int. Cl.5: **C12M 1/00**

(21) Numéro de dépôt: **85400349.8**

(22) Date de dépôt: **25.02.85**

(54) **Procédé et installation pour réaliser la dégradation en milieu anaérobie de produits, sous-produits et déchets organiques d'origine humaine, animale et/ou végétale.**

(43) Date de publication de la demande:
**03.09.86 Bulletin 86/36**

(45) Mention de la délivrance du brevet:
**22.04.92 Bulletin 92/17**

(84) Etats contractants désignés:
**AT BE CH DE GB IT LI LU NL SE**

(56) Documents cités:
FR-A- 893 537          FR-A- 1 146 820
FR-A- 2 305 113        FR-A- 2 466 176
FR-A- 2 506 178        FR-A- 2 510 605
FR-A- 2 551 457        GB-A- 2 074 997

(73) Titulaire: **VALORGA PROCESS SA**
**5, Rue de Massacan**
**F- Z.I. VENDARGUES 34740(FR)**

Titulaire: **Pavia, André**
**408 rue Valéry Larbaud**
**F-34100 Montpellier(FR)**

Titulaire: **Ducellier, Lucienne Vve. Ducellier, Gilbert**
**Collines d'Estanove Bâtiment D2 Route de Laverune**
**F-34000 Montpellier(FR)**

Titulaire: **Ducellier, Colette Epse. Segonzac, Louis**
**Parc d'Elancourt 9 rue de la Haie à Sorel**
**F-78190 Elancourt-Maurepas (Yveline)(FR)**

Titulaire: **Ducellier, Michèle Epse. Fabres, Jean**
**292 rue du Lavandin Les Collines d'Estanove**
**F-34000 Montpellier(FR)**

Titulaire: **Ducellier, Monique Epse. Goudet, Jean**
**Cité Rochambeau Entrée D2**
**F-06230 Villefranche sur Mer (Alpes Maritimes)(FR)**

Titulaire: **Ducellier, Véronique représentée par son tuteur légal Richert, Renée Vve Ducellier, Alain**
**29 avenue du Ried Lotissement des Bruyères**
**F-67800 Hoenheim(FR)**

(72) Inventeur: **Pavia, André**
**408 rue Valéry Larbaud**
**F-34100 Montpellier(FR)**
Inventeur: **Ducellier, Gilbert**
**décédé**
**(FR)**

(74) Mandataire: **Portal, Gérard et al**
**Cabinet Z. Weinstein 20, Avenue de Fried-**
**land**
**F-75008 Paris(FR)**

## Description

La présente invention a pour objet un procédé et une installation pour réaliser la dégradation en milieu anaérobie de produits, sous-produits et déchets organiques d'origine humaine, animale et/ou végétale. Plus particulièrement, l'invention a pour objet un procédé selon le préambule de la revendication 1.

Un procédé de ce type est connu par la demande de brevet européen EP-A-0074290 des demandeurs. Ce procédé décrit divers moyens pour obtenir une poussée pneumatique du substrat et une homogénéisation de celui-ci pour réaliser une circulation dirigée forcée et une bonne fluidisation de celui-ci.

Ces moyens comprennent l'alimentation ou l'évacuation des produits pneumatiquement de préférence à travers des siphons, par poussé pneumatique ; la provocation de variations brutales de pression de gaz contenu dans l'enceinte fermée en combinaison avec une réintroduction du biogaz dans la masse de produits présents dans l'enceinte.

On décrit également dans la demande de brevet européen EP-A-0074290 une insufflation de biogaz par jets brefs et successifs par des conduits débouchant dans le fond de la cuve de fermentation. On produisait ainsi une insufflation de gaz simultanée dans la totalité du fond de la cuve de fermentation par l'émission de jets brefs par intermittence. Il est maintenant apparu au cours de recherches complémentaires que cette insufflation simultanée dans la totalité de la cuve de fermentation à l'aide de jets brefs et successifs dans le temps n'aboutissait pas au résultat escompté de telle sorte que la fluidisation était souvent imparfaite. D'autre part, il a pu être constaté au cours de ces recherches que la densité du substrat varie depuis le puits d'alimentation jusqu'au puits d'évacuation de telle sorte qu'une injection uniforme de biogaz dans toute la cuve de fermentation est inadaptée en ce qu'elle ne tient pas du tout compte de cette différence de densité.

La présente invention a donc pour but d'éliminer les inconvénients du procédé connu.

Pour atteindre ce but, le procédé selon l'invention est caractérisé en ce que l'on subdivise virtuellement l'enceinte en une pluralité de secteurs de façon que la densité de produits correspondant respectivement à chacun des secteurs varie dans le sens de circulation des produits dans l'enceinte, et en ce qu'on injecte du biogaz produit dans chaque secteur successivement, de manière décalée dans le temps et sous une pression et pendant une période de temps prédéterminée, réglables sélectivement dans chaque secteur en fonction de la densité des produits dans le secteur associé en vue de réaliser une fluidisation satisfaisante des produits.

Ainsi, selon la présente invention, on réintroduit du biogaz dans chaque secteur successivement, c'est-à-dire de manière décalée dans le temps, de sorte à obtenir une rotation de l'injection de biogaz dans l'enceinte, d'un secteur à l'autre, cette rotation pouvant être régulière ou irrégulière et on adapte la pression d'injection du biogaz et la durée de l'injection en fonction de la densité actuelle des produits solides ou du substrat dans le secteur d'injection. On comprend donc aisément qu'on obtient avec l'invention une fluidisation parfaite des produits au cours de leur circulation dans la cuve de fermentation.

Selon une caractéristique du procédé selon l'invention, on prévoit un réservoir de stockage de biogaz sous haute pression en amont de l'enceinte de fermentation, la pression étant au moins égale à la pression d'injection dans le secteur où la densité des produits est la plus élevée.

Egalement avantageusement, selon le procédé de l'invention, l'injection de biogaz est programmée. Cette programmation peut être réalisée par tout moyen connu de l'homme du métier et en particulier par microprocesseur ou par ordinateur.

Selon encore une autre caractéristique du procédé de l'invention, constituant une variation de réalisation particulièrement avantageuse, on effectue une introduction directe dans l'enceinte des produits à dégrader, de préférence vers le fond de ladite enceinte à une distance prédéterminée dudit fond. De préférence, cette introduction directe est réalisée par poussée mécanique.

On obtient ainsi une simplification du procédé et de l'installation en évitant la présence de puits d'entrée qui oblige à travailler dans l'enceinte à une pression supérieure à la pression atmosphérique.

En effet, avec une introduction directe dans l'enceinte selon l'invention, en particulier par poussée mécanique, il est possible de travailler de façon anaérobie à la pression atmosphérique ce qui évite les surcoûts dûs à la fabrication de cuves capables de résister à des pressions de service supérieures à 1 bar.

Cette poussée mécanique peut être réalisée de manière particulièrement simple par une pompe à matière épaisse, pouvant être de préférence à piston ou à vis.

Selon encore une autre caractéristique du procédé selon l'invention, on réalise une agitation au biogaz immédiatement à l'orifice d'entrée des produits dans l'enceinte.

L'invention a également pour objet une installation pour la mise en oeuvre du procédé, du type comprenant un réacteur ayant une cuve de fermentation anaérobie divisée verticalement en deux parties, une première partie étant reliée à l'entrée des produits dans ladite cuve, la seconde partie étant reliée à la sortie des produits fermentée, une sortie de biogaz reliée à l'entrée d'un dispositif formant source de biogaz sous pression, la sortie de ce dispositif étant reliée à une pluralité d'orifices dans le fond de la cuve par l'intermédiaire de moyens de vannes, caractérisée en ce que les orifices précités sont disposés dans le fond de la cuve dans des zones correspondant aux secteurs de densité précités, se succédant dans le sens de circulation des produits dans ladite enceinte, en ce que les orifices de chaque zone sont reliés par une vanne commune sélectivement réglable à ladite source de biogaz sous pression, et en ce que l'installation comprend un dispositif de commande programmée sélective adaptée pour commander l'instant et la durée d'ouverture de chaque vanne, et la pression du gaz à injecter.

Selon une caractéristique avantageuse de l'installation selon l'invention, le circuit d'injection du biogaz comprend un caisson de stockage de biogaz sous pression dans lequel le biogaz est emmagasiné jusqu'à l'obtention d'une pression au moins égale à la pression la plus élevée requise dans l'un des secteurs de la cuve.

Selon un autre mode de réalisation particulièrement avantageux de l'installation selon l'invention, celle-ci comprend un conduit d'injection du biogaz au sommet de la cuve de fermentation pourvu d'une vanne appropriée, afin de réaliser une poussée pneumatique dans la cuve de fermentation avantageusement réalisée simultanément à l'extraction de produits de la cuve de fermentation par exemple en direction du puits d'évacuation.

Selon une autre caractéristique de l'installation selon l'invention, constituant une variante de réalisation particulièrement avantageuse, ladite installation comprend des moyens d'introduction directe dans l'enceinte des produits à dégrader, de préférence vers le fond de ladite enceinte à une distance prédéterminée dudit fond. Selon un mode de réalisation particulier, ces moyens d'introduction directe comprennent des moyens réalisant une poussée mécanique, de préférence comprenant une pompe à matière épaisse.

Selon un mode de réalisation particulier, cette pompe à matière épaisse peut être une pompe à piston ou une pompe à vis.

Selon encore une autre caractéristique de l'installation selon l'invention, celle-ci comprend un tronçon de raccordement à l'orifice d'entrée de l'enceinte entre les moyens d'introduction directe et l'orifice proprement dit, assurant une étanchéité.

Selon une autre caractéristique de l'installation selon l'invention, celle-ci comprend des moyens d'agitation au biogaz immédiatement ou au voisinage de l'orifice d'entrée des produits dans l'enceinte.

Selon une autre caractéristique de l'installation selon l'invention, la sortie des produits dégradés dans l'enceinte est une sortie tangentielle permettant une éjection par gravité.

Selon encore une autre caractéristique de l'installation selon l'invention, celle-ci comprend un dispositif divergent à l'entrée des produits, de dimension appropriée afin de faciliter l'entrée des produits dans l'enceinte de fermentation. Egalement, il est préféré que la sortie soit également équipée d'un dispositif convergent facilitant la sortie des produits fermentés ou digestat.

D'autres buts, caractéristiques et avantages de l'invention apparaîtront clairement à la leture de la description explicative qui va suivre faite en référence aux dessins annexés dans lesquels :

La figure 1 représente schématiquement une installation montrant en coupe axiale la cuve de fermentation représentée à la figure 2 mais avec les puits d'alimentation et d'évacuation des produits diamétralement opposés pour plus de clarté alors qu'à la figure 2 ils sont représentés côte à côte.

La figure 2 représente une vue en coupe transversale et de dessus du fond de la cuve de fermentation.

La figure 3 représente une variante de réalisation pour introduction directe des produits dans la cuve.

La figure 4 représente une coupe axiale de la figure 3, et

La figure 5 représente une variante de réalisation de réintroduction du biogaz.

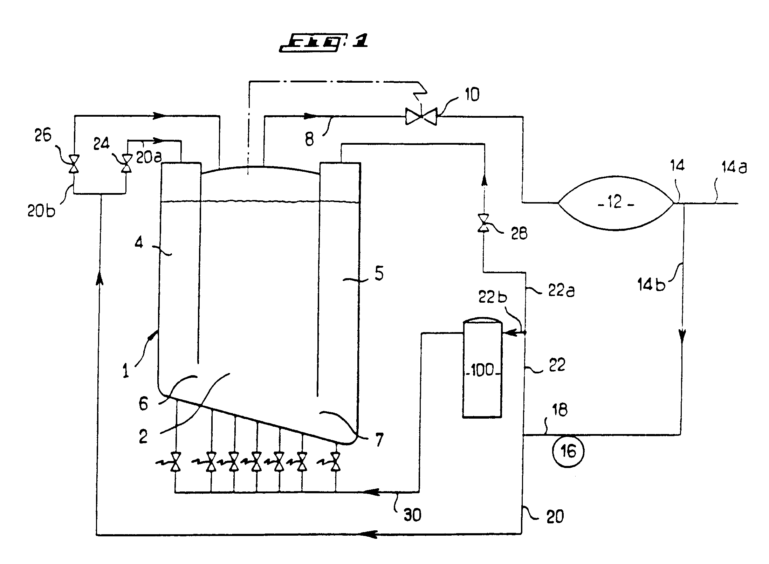

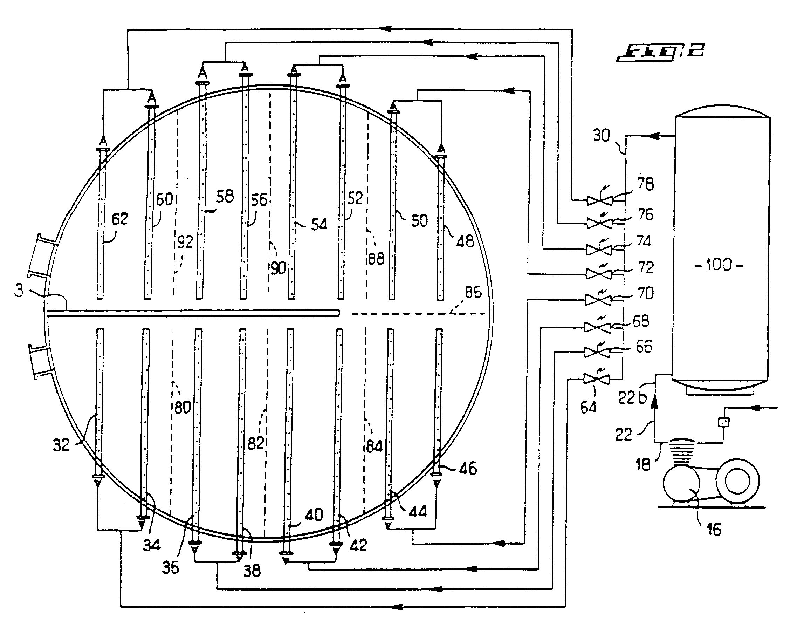

En référence aux figures 1 et 2, une installation selon l'invention comprend un réacteur 1 ayant une cuve 2 de fermentation anaérobie divisée verticalement par une cloison transversale 3 en deux parties, une première partie étant reliée à un puits d'alimentation 4 et une deuxième partie étant reliée à un puits d'évacuation 5 à travers respectivement un premier siphon 6 et un second siphon 7, conformément à la demande de brevet européen EP-A-0074 290.

Cette installation comprend également une sortie de gaz ou biogaz formée par un conduit 8 comportant une vanne 10 d'évacuation du biogaz, ledit conduit 8 étant relié par l'intermédiaire d'une vanne 10 à un récipient 12 formant gazomètre et dont la sortie 14 se subdivise en deux conduits 14a et 14b, le conduit 14a étant relié à un dispositif de consommation de biogaz qui peut être par exemple un brûleur, etc. Le conduit de dérivation 14b est pourvu d'un compresseur 16 dont la sortie 18 se subdivise en plusieurs conduits de dérivation 20, 22. Le conduit de dérivation 20 alimente par exemple encore par une subdivision deux vannes indépendantes 24, 26 commandant l'injection du biogaz respectivement vers le sommet du puits d'alimentation 4 et vers le sommet de la cuve de fermentation 2. L'autre conduit de subdivision 22 se subdivise à son tour pour alimenter en biogaz directement du compresseur 16 par la dérivation 22a le sommet du puits d'évacuation 5 par l'intermédiaire d'une vanne 28. Une autre subdivision 22b du conduit 22a est reliée par une conduite 30 à une pluralité de conduits que l'on voit clairement à la figure 2 numérotés respectivement 32, 34, 36, 38, 40, 42, 44, 46, 48, 50, 52, 54, 56, 58, 60, 62 débouchant dans le fond de la cuve 2 de fermentation et constitués par des tubes de section appropriée pourvus longitudinalement d'une pluralité d'orifices d'injection. Chaque conduit comporte une vanne associée.

Selon la présente invention, cette pluralité de conduits 32 à 62 est alimentée en biogaz individuellement ou en groupes (ici deux par deux), comme cela se voit bien à la figure 2, par des vannes indépendantes 64, 66, 68, 70, 72, 74, 76, 78 de manière à subdiviser la cuve en secteurs indépendamment alimentés en biogaz. Les secteurs sont ici matérialisés symboliquement par la présence des lignes pointillées 80, 82, 84, 86, 88, 90, 92 que l'on voit bien à la figure 2. On notera que les conduits de réinjection de biogaz dans la cuve sont latéralement espacés entre eux et disposés au moins dans la majorité de la cuve, de préférence de façon régulière comme présenté. La forme des conduits 32 - 62 fait partie de l'invention.

Selon la présente invention, chaque vanne 64 à 78 est prévue de manière à délivrer une pression fonction de la densité des produits dans le secteur associé. D'autre part, chaque vanne est commandée de préférence selon l'invention par un dispositif de commande programmée (non représenté), de telle sorte qu'on injecte par intermittence du biogaz dans chacun des secteurs de la cuve 2 sous pression et pendant une période de temps prédéterminée, réglables en fonction de la densité des produits dans son secteur associé.

Egalement selon un mode de réalisation avantageux de l'invention, le circuit d'injection de biogaz (conduits 22, 30) comprend un caisson 100 de stockage de biogaz sous pression dans lequel le biogaz est emmagasiné jusqu'à obtention d'une pression au moins égale à la pression la plus élevée requise dans l'un des secteurs de la cuve (2).

Par pression d'injection en fonction de la densité des produits dans un secteur donné, on entend que la pression d'injection est suffisante pour produire une fluidisation de la masse des produits ayant ladite densité.

On notera que la présence de ce réservoir de stockage 100 de biogaz sous pression élevée permet de manière inattendue d'utiliser un compresseur 16 de faible capacité, c'est-à-dire de faible coefficient de compression.

On notera par ailleurs que selon la présente invention, par le conduit 20b de réinjection de biogaz par l'intermédiaire de la vanne 26, on peut réaliser une poussée pneumatique au sommet de la cuve 2 de manière à évacuer les produits solides circulant sur le fond du réacteur vers le puits d'évacuation 5.

Egalement on notera que la vanne 10 d'évacuation du biogaz dans la cuve 2 est une vanne quelconque. Il peut s'agir d'une vanne commandée hydrauliquement, pneumatiquement ou électriquement ou même une vanne ou valve hydraulique.

Ainsi, cette installation permet de réaliser le procédé précédemment indiqué, à savoir qu'on obtient une subdivision de la cuve 2, ou enceinte de fermentation fermée, en une pluralité de secteurs. En vue de réaliser une fluidisation parfaite ou homogène des produits solides ou substrats dans la cuve 2, on injecte par intermittence du biogaz dans chacun des secteurs sous une pression et pendant une période de temps prédéterminées, réglables en fonction de la densité des produits dans ledit secteur associé.

De même, selon une caractéristique avantageuse de l'invention, on injecte du biogaz dans chaque secteur successivement de manière décalée dans le temps de sorte à obtenir une rotation de l'injection de biogaz dans l'enceinte d'un secteur à l'autre depuis le puits d'alimentation 4 jusqu'au puits d'évacuation 5. De même, et grâce au dispositif de programmation, on obtient une injection programmée. Cette programmation peut d'ailleurs être asservie automatiquement à des moyens de détection d'un manque de fluidité dans l'un des secteurs de la cuve 2 de manière à commander immédiatement une injection dans ledit secteur.

Selon une autre caractéristique de l'invention, on effectue la poussée pneumatique dans le puits d'alimentation 4 simultanément à la commande de l'ouverture de la vanne 10 d'évacuation de biogaz produit dans la cuve 2 et de même on effectue la poussée pneumatique dans le puits d'évacuation 5 simultanément à l'injection de biogaz par la vanne 26 au sommet de la cuve 2.

Ainsi selon l'invention, l'obtention d'une fluidisation pratiquement parfaite des produits ou substrats même à teneur élevée en matière solide comme le fumier ou les déchets urbains, permet une simplification de la technologie de la fabrication du fond de la cuve de fermentation en permettant par exemple d'en diminuer la pente ce qui se traduit par un coût de cuverie moins élevé.

D'autre part, on obtient naturellement un meilleur rendement en biogaz.

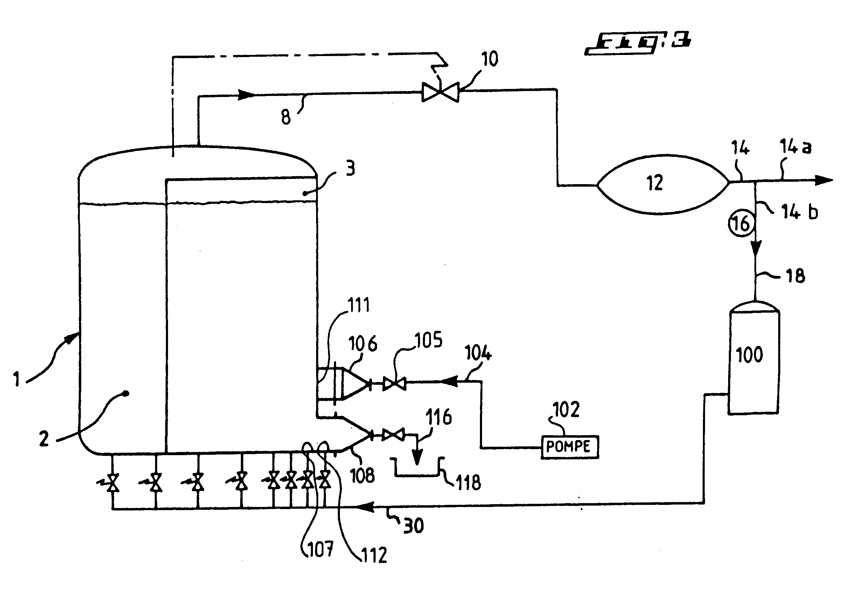

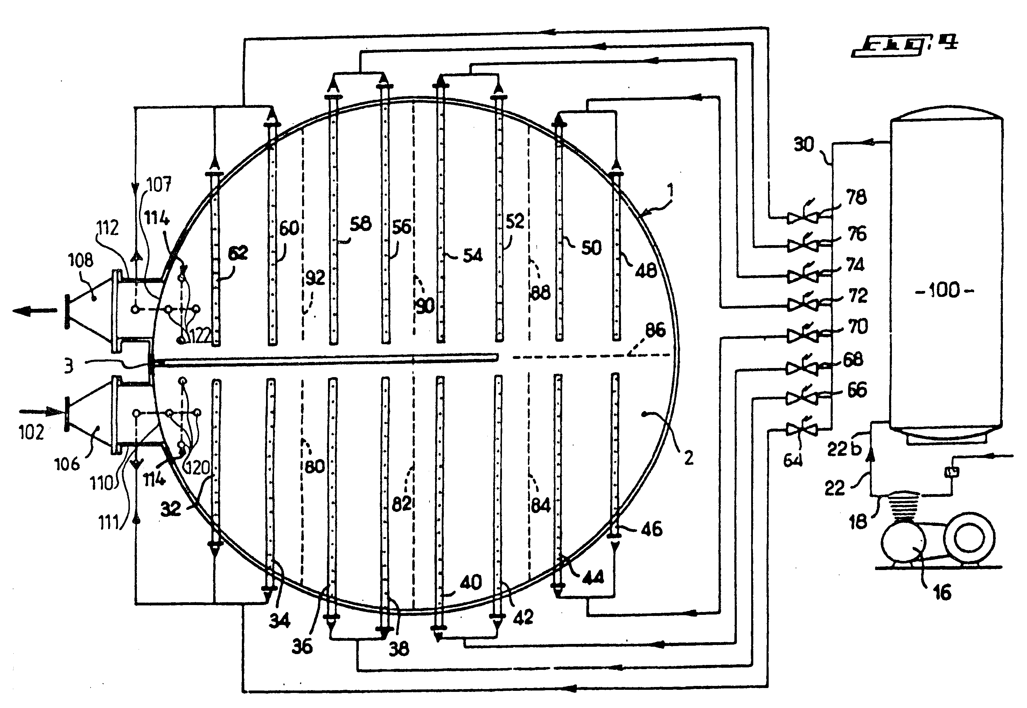

Selon une variante de réalisation faisant l'objet des figures 3 et 4, l'installation selon l'invention comprend des moyens 102 d'introduction directe des produits à dégrader par fermentation anaérobie, de préférence vers le fond de l'enceinte. Ces moyens d'introduction directe comprennent de préférence des moyens d'introduction par poussée mécanique, avantageusement comprenant au moins une pompe à matière épaisse 102 des produits fermentés.

De telles pompes à matière épaisse sont actuellement disponibles sur le marché et peuvent être constituées par exemple par des pompes à béton. De préférence, la pompe à matière épaisse qui est utilisée est à piston ou à vis.

Selon cette variante de réalisation, la pompe 102 alimente par une conduite 104 pouvant être pourvue d'une vanne 105 de préférence un tronçon de raccordement 110 communiquant avec l'orifice d'entrée 111 proprement dit dans la cuve ou enceinte 2.

La présence de ce tronçon de raccordement permet d'assurer une étanchéité parfaite entre les moyens d'introduction directe 102 et l'intérieur de la cuve 2.

Selon cette variante de réalisation, l'orifice 111 est disposé à une distance prédéterminée du fond de la cuve 2.

D'autre part, l'orifice de sortie 107 des produits dégradés depuis l'enceinte est de préférence réalisé tangentiellement au fond de la cuve de manière à assurer une sortie des produits dégradés par gravité.

Il est à noter que selon la présente invention, le fond de la cuve peut être en pente ou sensiblement horizontal compte tenu que la circulation des produits dans la cuve ou enceinte 2 est assurée par les moyens d'introduction directe qui réalisent une introduction sous pression grâce à la poussée mécanique.

De même, on prévoit un tronçon de raccordement 112 à la sortie 107 de manière à assurer également l'étanchéité entre l'extérieur et l'intérieur de l'enceinte 2 afin de ne pas perturber la fermentation se produisant à l'intérieur de l'enceinte 2.

Selon une caractéristique particulièrement avantageuse de la présente invention, on interpose entre les moyens d'introduction 102 et l'entrée 111 de la cuve 2, de préférence entre les moyens 102 et le tronçon 110 de raccordement, un dispositif divergent 106, de préférence de forme tronconique dont la grande base est raccordée au tronçon de raccordement 110, de dimension appropriée afin de faciliter l'entrée des produits dans l'enceinte ou cuve de fermentation et favoriser du fait du front de poussée, une avancée progressive de la matière présente dans l'enceinte.

De même, de préférence, on dispose à la sortie des produits de l'enceinte 2, un dispositif convergent 108 qui favorise la sortie du digestat (matières fermentées), par exemple dans une tuyauterie de récupérateur 116, (figure 3) et sa jetée vers une presse symbolisée par le numéro de référence 118.

On notera que selon cette variante de réalisation, la distribution et la subdivision du biogaz est réalisée comme dans le mode de réalisation faisant l'objet des figures 1 et 2.

Selon une caractéristique préférée, on prévoit également des moyens d'agitation 114 à l'entrée et de préférence également à la sortie de la cuve 2 de manière à réaliser une agitation au biogaz conforme en volume et en temps avec la densité du produit à dégrader. Cette agitation peut être réalisée immédiatement ou à proximité de l'orifice d'entrée 111 ou de sortie 107.

Ces agitations sont réalisées conformément au cycle décrit en référence aux figures 1 et 2 et pourront donc être régulièrement répétées ou non, avec un volume de gaz bien adapté et ce par l'intermédiaire du compresseur 16 et du réservoir tampon 100.

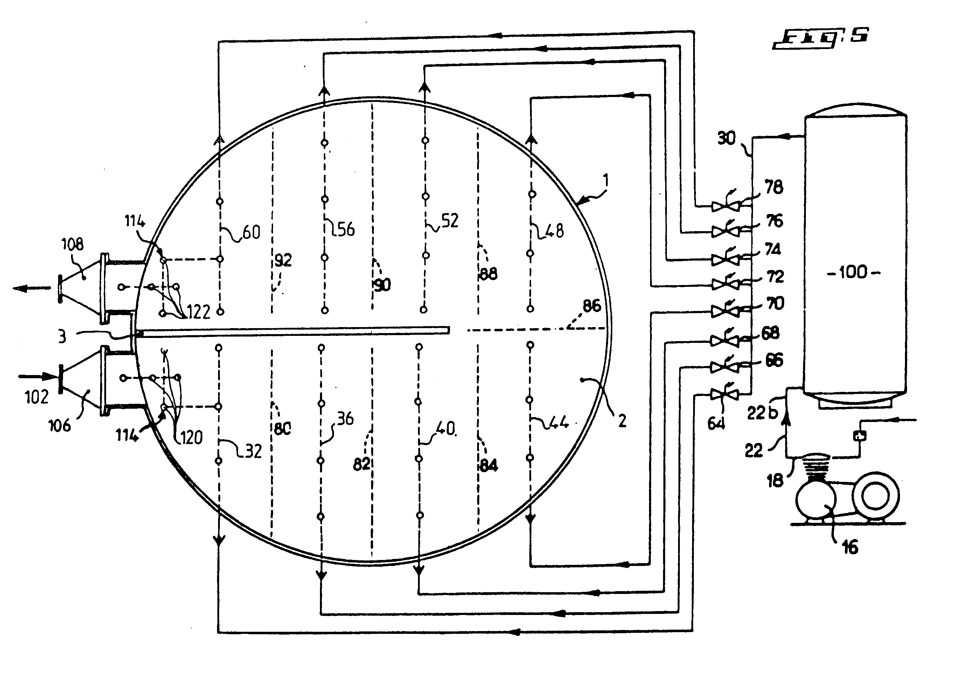

Selon la variante de réalisation faisant l'objet de la figure 5, les moyens d'agitation 114 sont réalisés en dérivation du conduit de réintroduction de biogaz 32 ou 60, cette dérivation étant interne à l'enceinte 2. Cette dérivation peut comporter plusieurs orifices de sortie de biogaz 120 ou 122 alors que selon le mode de réalisation de la figure 4, cette dérivation est réalisée à l'extérieur de la cuve 2.

Egalement, selon le mode de réalisation de la figure 5, chaque secteur indépendamment alimenté en biogaz peut ne comprendre qu'un conduit de réintroduction de biogaz 32, 36, 40, 44, 48, 52, 56, 60. Naturellement, comme pour le mode de réalisation de la figure 4, on peut prévoir deux ou davantage conduits de réintroduction de biogaz.

On comprendra ainsi que cette installation fonctionne selon le procédé précédemment décrit et qu'on obtient une amélioration du procédé, comprenant une amélioration de l'installation tout en réalisant une fluidisation parfaite et homogène des produits solides ou substrats dans la cuve 2.

D'autre part, selon la variante des figures 3 à 5, on obtient une introduction directe des produits en permettant de travailler de façon anaérobie à la pression atmosphérique tout en évitant les surcoûts dûs à la fabrication de cuves capables de résister à des pressions de service supérieures à 1 bar, et en résolvant simultanément le problème d'étanchéité, en particulier par la présence de tronçons de raccordement formant un bouchon de matière étanche permettant de ne pas perturber la fermentation.

## Revendications

1. Procédé pour réaliser une dégradation en milieu anaérobie, par exemple une méthanogénèse, de produits, sous-produits ou déchets organiques d'origine humaine, animale et/ou végétale consistant à introduire lesdits produits à dégrader dans une enceinte fermée, après avoir éventuellement ensemencé lesdits produits avec un substrat convenable, à imposer auxdits produits un sens de circulation dans ladite enceinte, à recueillir le gaz produit appelé biogaz dégagé au-dessus de ladite masse de produits et à évacuer les produits dégradés, avec une réintroduction intermittente de biogaz sous forme de jets à partir du fond de l'enceinte dans la masse de produits présente dans l'enceinte, caractérisé en ce que l'on subdivise virtuellement ladite enceinte en une pluralité de secteurs de façon que la densité de produits correspondant respectivement à chacun des secteurs varie dans le sens de circulation des produits dans ladite enceinte, et en ce qu'on injecte du biogaz produit dans chaque secteur successivement, de manière décalée dans le temps et sous une pression et pendant une période de temps prédéterminées, réglables sélectivement dans chaque secteur en fonction de la densité des produits dans le secteur associé en vue de réaliser une fluidisation satisfaisante des produits.

2. Procédé selon la revendication 1, caractérisé en ce qu'on injecte du biogaz produit dans chaque secteur virtuellement défini sous forme d'une pluralité de jets de biogaz simultanément dans le même secteur.

3. Procédé selon la revendication 1 ou 2, caractérisé en ce qu on prévoit un réservoir de stockage de biogaz sous haute pression en amont de l'enceinte de fermentation, la pression étant au moins égale à la pression d'injection dans le secteur de densité la plus élevée donc de fluidité la plus faible.

4. Procédé selon l'une des revendications 1 à 3, caractérisé en ce que l'injection est programmée, de préférence en étant également asservie automatiquement à la détection d'un manque de fluidité.

5. Procédé selon l'une des revendications 1 à 4, caractérisé en ce qu'on effectue la poussée pneumatique à l'entrée de l'enceinte simultanément à la commande de l'ouverture de la vanne d'évacuation de biogaz produit dans l'enceinte tandis qu'on effectue la poussée pneumatique à la sortie de l'enceinte simultanément à l'injection de biogaz au sommet de l'enceinte.

6. Procédé selon l'une quelconque des revendications 1 à 4, caractérisé en ce qu'on effectue une introduction directe dans l'enceinte des produits à dégrader, de préférence vers le fond de ladite enceinte à une distance prédéterminée dudit fond.

7. Procédé selon la revendication 6, caractérisé en ce qu'on réalise une introduction directe par poussée mécanique, tandis que la sortie est de préférence réalisé par gravité.

8. Procédé selon la revendication 7, caractérisé en ce que la poussée mécanique est réalisée par une pompe à matière épaisse, de préférence à piston ou à vis.

9. Procédé selon l'une des revendications 6 à 8, caractérisé en ce qu'on réalise une agitation au biogaz immédiatement ou au voisinage des orifices d'entrée ou de sortie des produits dans ou hors de l'enceinte.

10. Procédé selon l'une quelconque des revendications précédentes, caractérisé en ce qu'on injecte du biogaz dans chaque secteur successivement, de manière décalée dans le temps, de sorte à obtenir une rotation régulière ou irrégulière de l'injection de biogaz dans l'enceinte d'un secteur à l'autre.

11. Installation pour la mise en oeuvre du procédé selon l'une des revendications précédentes du type comprenant un réacteur ayant une cuve de fermentation anaérobie (2) divisée verticalement en deux parties, une première partie étant reliée à l'entrée des produits dans ladite cuve, la seconde partie étant reliée à la sortie des produits fermentés, une sortie de biogaz reliée à l'entrée d'un dispositif formant source de biogaz sous pression, la sortie de ce dispositif étant reliée à une pluralité d'orifices dans le fond de la cuve par l'intermédiaire de moyens de vanne, caractérisée en ce que les orifices précités sont disposés dans le fond de la cuve dans des zones correspondant aux secteurs de densité précités, se succédant dans le sens de circulation des produits dans ladite enceinte, en ce que les orifices de chaque zone sont reliés par une vanne commune sélectivement réglable à ladite source de biogaz sous pression, et en ce que l'installation comprend un dispositif de commande programmée sélective adapté pour commander l'instant et la durée d'ouverture de chaque vanne, et la pression du gaz à injecter.

12. Installation selon la revendication 11, caractérisée en ce que le dispositif formant source de biogaz sous pression comprend un compresseur (16) de faible capacité relié par sa sortie à un caisson (100) de stockage du biogaz sous haute pression dans lequel le gaz peut être emmagasiné sous une pression au moins égale à la pression la plus élevée requise dans l'un des secteurs de la cuve (2).

13. Installation selon l'une des revendications 11 ou 12, caractérisée en ce que le circuit d'injection de biogaz comprend un conduit (20b) d'injection de biogaz au sommet de la cuve (2) de fermentation comportant une vanne propre (26) pour réaliser une poussée pneumatique dans la cuve (2) de fermentation.

14. Installation selon l'une des revendications 11 à 13, caractérisée en ce qu'elle comprend des moyens (102) d'introduction directe des produits à dégrader dans l'enceinte, de préférence vers le fond de l'enceinte et à distance prédéterminée dudit fond.

15. Installation selon la revendication 14, caractérisée en ce que les moyens d'introduction directe (102) comprennent des moyens réalisant une poussée mécanique de préférence comprenant au moins une pompe à matière épaisse, avantageusement à piston ou à vis.

16. Installation selon la revendication 14 ou 15, caractérisée en ce qu'elle comprend un tronçon de raccordement entre les moyens d'introduction directe (102) et l'entrée (111) de l'enceinte (2).

17. Installation selon l'une des revendications 14 à 16, caractérisée en ce qu'elle comprend des moyens d'agitation (114) immédiatement ou au voisinage de l'entrée (111) et avantageusement de la sortie (107) de l'enceinte.

18. Installation selon l'une des revendications 14 à 17, caractérisée en ce que l'orifice de sortie des produits est tangentielle au fond de l'enceinte.

19. Installation selon l'une des revendications 14 à 18, caractérisée en ce que l'entrée de l'enceinte est pourvue d'un dispositif divergent (106) tandis qu'avantageusement la sortie est pourvue d'un dispositif convergent (108), facilitant respectivement l'entrée et la sortie des produits relativement à l'enceinte (2.)

**Claims**

1. Method of carrying out a degradation in an anaerobic medium, for instance a methanogenesis, of organic products, by-products or waste of human, animal and/or vegetable origin consisting in feeding the said products to be degraded into a closed vessel after having possibly seeded said products with a suitable substrate, imposing upon the said products a direction of circulation within the said vessel, recovering the gas produced called biogas evolved above the said body of products and discharging the degraded products, with an intermittent re-introduction of the biogas as jets from the bottom of the vessel into the mass of products present within the vessel, characterized in that one virtually subdivides the said vessel into a plurality of sectors so that the density of products corresponding to each one of the sectors, respectively, varies in the direction of circulation of the products within the said vessel and in that one injects produced biogas into each sector successively in a time-shifted manner and under a pressure and for a period of time which are predetermined, selectively adjustable in each sector in accordance with the density of the products in the associated sector with a view to perform a satisfactory fluidizing of the products.

2. Method according to claim 1, characterized in that one injects produced biogas in each virtually defined sector as a plurality of jets of biogas simultaneously in the same sector.

3. Method according to claim 1 or 2, characterized in that one provides a tank for storing biogas under high pressure upstream of the fermentation vessel, the pressure being at least equal to the injection pressure in the sector with the highest density hence the smallest fluidity.

4. Method according to one of claims 1 to 3, characterized in that the injection is programmed preferably by being also automatically interlocked with the detection of a lack of fluidity.

5. Method according to one of claims 1 to 4, characterized in that one effects the pneumatic thrust at the inlet of the vessel at the same time as the actuation of the opening of the valve for discharging the biogas produced in the vessel whereas one effects the pneumatic thrust at the outlet of the vessel at the same time as the injection of biogas at the top of the vessel.

6.　Method according to any one of claims 1 to 4, characterized in that one effects a direct introduction into the vessel of products to be degraded preferably towards the bottom of the said vessel at a predetermined distance from the said bottom.

7.　Method according to claim 6, characterized in that one carries out a direct introduction through mechanical thrust whereas the egress is preferably carried out through gravity.

8.　Method according to claim 7, characterized in that the mechanical thrust is produced by a pump for thick material, preferably of the piston or screw type.

9.　Method according to one of claims 6 to 8, characterized in that one performs a stirring with biogas immediately at or in the vicinity of the inlet or outlet ports of the products into or out of the vessel.

10.　Method according to any one of the foregoing claims, characterized in that one injects biogas into each sector successively in a time-shifted manner so as to obtain a regular or irregular rotation of the injection of biogas within the vessel from one sector to another one.

11.　Equipment for carrying out the method according to one of the foregoing claims of the type comprising a reactor having an anaerobic fermentation vat (2) divided vertically into two parts, a first part being connected to the inlet of the products into the said vat, the second part being connected to the outlet of the fermented products, a biogas outlet connected to the inlet of a device forming a source of biogas under pressure, the outlet of this device being connected to a plurality of ports in the bottom of the vat through the medium of valve means, characterized in that the aforesaid ports are disposed in the bottom of the vat within zones corresponding to the aforesaid density sectors, following one another in the direction of circulation of the products within the said vessel, in that the ports of each zone are connected by a selectively adjustable common valve to the said source of biogas under pressure and in that the equipment comprises a programmed selective control device adapted to operatively set the time and the duration of opening of each valve and the pressure of the gas to be injected.

12.　Equipment according to claim 11, characterized in that the device forming a source of biogas under pressure comprises a small capacity compressor (16) connected with its outlet to a container (100) for storing biogas under high pressure in which the gas may be stored under a pressure at least equal to the highest pressure required in one of the sectors of the vat (2).

13.　Equipment according to one of claims 11 or 12, characterized in that the biogas injection circuit comprises a biogas injection duct (20b) at the top of the fermentation vat (2) comprising a valve proper (26) for producing une pneumatic thrust in the fermentation vat (2).

14.　Equipment according to one of claims 11 to 13, characterized in that it comprises means (102) for the direct introduction of the products to be degraded into the vessel, preferably towards the bottom of the vessel and at a predetermined distance from the said bottom.

15.　Equipment according to claim 14, characterized in that the direct introduction means (102) comprise means for producing a mechanical thrust preferably comprising at least one pump for thick material advantageously of the piston or screw type.

16.　Equipment according to claim 14 or 15, characterized in that it comprises a connecting section between the direct introduction means (102) and the inlet (111) of the vessel (2).

17.　Equipment according to one of claims 14 to 16, characterized in that it comprises stirring means (114) immediately at or in the vicinity of the inlet (111) and advantageously of the outlet (107) of the vessel.

18.　Equipment according to one of claims 14 to 17, characterized in that the outlet port for the products is tangential to the bottom of the vessel.

19.　Equipment according to one of claims 14 to 18, characterized in that the inlet of the vessel is provided with a divergent device (106) whereas advantageously the outlet is provided with a convergent device (108) facilitating the ingress and the egress of the products, respectively, in relation to the vessel (2).

**Patentansprüche**

1.　Verfahren zur Durchführung eines Abbaues in anaerobischem Medium von organischen Erzeugnissen, Nebenerzeugnissen oder Abfällen menschlicher, tierischer und/oder pflanzlicher Herkunft, zum Beispiel einer Methanentstehung, das darin besteht, die besagten abzubauenden Erzeugnisse in ein geschlossenes Gefäss einzuführen, nachdem man gegebenenfalls die besagten Erzeugnisse mit einem geeigneten Substrat eingeimpft hat, den besagten Erzeugnissen eine Umlaufrichtung in dem besagten Gefäss aufzuzwingen, das über der besagten Masse von Erzeugnissen freigesetzte Biogas genannte Gas aufzufangen und die abgebauten Erzeugnisse abzuführen, bei intermittierender Wiedereinführung des Biogases als Strahlenvon dem Boden des Gefässes aus in die in dem Gefäss vorhandene Masse von Erzeugnissen, dadurch gekennzeichnet, dass man das besagte Gefäss in eine Vielzahl von Sektoren virtuell unterteilt, so dass die jeweils jedem der Sektore entsprechende Dichte der Erzeugnissen sich in der Umlaufrichtung der Erzeugnissen in dem besagten Gefäss verändert und dass man erzeugtes Biogas aufeinanderfolgend in jeden Sektor in zeitlich versetzter Weise und unter einem Druck und während eines Zeitraums, die vorbestimmt und in jedem Sektor in Abhängigkeit der Dichte der Erzeugnisse in dem zugeordneten Sektor wahlweise einstellbar sind, einspritzt, um eine zufriedenstellende Fluidisierung der Erzeugnisse zustandezubringen.

2. Verfahren nach Anspruch 1, dadurch gekennzeichnet, dass man in jedem virtuell bestimmten Sektor erzeugtes Biogas als eine Vielzahl von Biogasstrahlen gleichzeitig in denselben Sektor einspritzt.

3. Verfahren nach Anspruch 1 oder 2, dadurch gekennzeichnet, dass man einen Behälter zur Speicherung von Biogas unter hohem Druck stromaufwärts des Gärgefässes vorsieht, wobei der Druck wenigstens dem Einspritzdruck in dem Sektor mit der grössten Dichte also mit der niedrigsten Fluidität gleich ist.

4. Verfahren nach einem der Ansprüche 1 bis 3, dadurch gekennzeichnet, dass die Einspritzung programmiert und vorzugsweise auch in Abhängigkeit des Nachweises eines Fluiditätmangels selbsttätig nachstellbar ist.

5. Verfahren nach einem der Ansprüche 1 bis 4,dadurch gekennzeichnet, dass man den pneumatischen Schub am Eintritt des Gefässes gleichzeitig mit der Steuerung der Oeffnung des Ventils zur Abführung des in dem Gefäss erzeugten Biogases durchführt, während man den pneumatischen Schub am Austritt des Gefässes gleichzeitig mit der Biogaseinspritzung an der Oberseite des Gefässes durchführt.

6. Verfahren nach irgendeinem der Ansprüche 1 bis 4, dadurch gekennzeichnet, dass man eine unmittelbare Einführung der abzubauenden Erzeugnisse in das Gefäss vorzugsweise im Bereich des Bodens des besagten Gefässes in einem vorbestimmten Abstand vom besagten Boden durchführt.

7. Verfahren nach Anspruch 6, dadurch gekennzeichnet, dass man eine unmittelbare Einführung durch mechanischen Schub durchführt, während der Austritt vorzugsweise durch die Schwerkraft geschieht.

8. Verfahren nach Anspruch 7, dadurch gekennzeichnet, dass der mechanische Schub durch eine Dickstoffpumpe, vorzugsweise der Kolben-bzw. Schneckenbauart durchführt.

9. Verfahren nach einem der Ansprüche 6 bis 8, dadurch gekennzeichnet, dass man ein Rühren mit Biogas unmittelbar an den Oeffnungen zum Eintritt bzw. Austritt der Erzeugnisse in das bzw. aus dem Gefäss oder im Bereich derselben durchführt.

10. Verfahren nach irgendeinem der vorangehenden Ansprüche, dadurch gekennzeichnet, dass man Biogas aufeinanderfolgend in jeden Sektor in zeitlich versetzter Weise einspritzt, um ein gleichmässiges oder ungleichmässiges Drehen der Biogaseinspritzung in dem Gefäss von einem Sektor zum anderen zu erzielen.

11. Anlage zur Durchführung des Verfahrens nach einem der vorangehenden Ansprüche, derjenigen Gattung, mit einem Reaktor mit einem senkrecht in zwei Teilen unterteilten Wanne (2) zur anaerobischen Gärung, wobei ein erstes Teil mit dem Eintritt der Erzeugnisse in die besagte Wanne verbunden ist, wobei das zweite Teil mit dem Austritt der gegärten Erzegnisse verbunden ist,einem mit dem Eintritt einer eine Biogasquelle unter Druck bildenden Vorrichtung verbundenen Biogasaustritt, wobei der Austritt dieser Vorrichtung mit einer Vielzahl von Oeffnungen in dem Boden der Wanne über Ventilmittel verbunden ist, dadurch gekennzeichnet, dass die vorgenannten Oeffnungen in dem Boden der Wanne in den vorgenannten Dichtesektoren entsprechenden, in der Umlaufrichtung der Erzeugnisse in dem besagten Gefäss aufeinanderfolgenden Bereichen angeordnet sind, dass die Oeffnungen jedes Bereiches durch ein wahlweise einstellbares gemeinsames Ventil mit der besagten Biogasquelle unter Druck verbunden sind und dass die Anlage eine an die Steuerung des Zeitpunktes und der Dauer der Oeffnung jedes Ventils und des Druckes des einzuspritzenden Gases angepasste Vorrichtung zur programmierten wahlweisen Steuerung aufweist.

12. Anlage nach Anspruch 11, dadurch gekennzeichnet, dass die eine Biogasquelle unter Druck bildende Vorrichtung einen Verdichter (16) schwacher Leistungsfähigkeit aufweist, der mit seinem Austritt an einen Behälter (100) zur Speicherung des Biogases unter hohem Druck verbunden ist, in welchem das Gas unter einem Druck gespeichert werden kann, der wenigstens dem in einem der Sektoren der Wanne (2) erforderlichen höchsten Druck gleich ist.

13. Anlage nach einem der Ansprüche 11 oder 12, dadurch gekennzeichnet, dass der Biogaseinspritzungskreislauf eine Leitung (20b) zur Einspritzung von Biogas an der ein eigenes Ventil (26) aufweisenden Oberseite der Gärwanne (2) aufweist, um einen pneumatischen Schub in der Gärwanne (2) zu erzeugen.

14. Anlage nach einem der Ansprüche 11 bis 13, dadurch gekennzeichnet, dass sie Mittel (102) zur unmittelbaren Einführung der abzubauenden Erzeugnisse in das Gefäss, vorzugsweise im Bereich des Bodens des Gefässes und in einer vorbestimmten Entfernung vom besagten Boden umfasst.

15. Anlage nach Anspruch 14, dadurch gekennzeichnet, dass die unmittelbaren Einführungsmittel (102) Mittel zur Durchführung eines mechanischen Schubes vorzugsweise mit wenigstens einer Dickstoffpumpe vorteilhaft der Kolben-bzw. Schneckenbauart aufweisen.

16. Anlage nach Anspruch 14 oder 15, dadurch gekennzeichnet, dass sie einen Verbindungsabschnitt zwischen den unmittelbaren Einführungsmitteln (102) und dem Eintritt (111) des Gefässes (2) aufweist.

17. Anlage nach einem der Ansprüche 14 bis 16, dadurch gekennzeichnet, dass sie Rührmittel (114) unmittelbar am Eingang (111) und vorteilhaft am Ausgang (107) des Gefässes oder im Bereich derselben aufweist.

18. Anlage nach einem der Ansprüche 14 bis 17, dadurch gekennzeichnet, dass die Austrittsöffnung für die Erzeugnisse tengential zum Boden des Gefässes ist.

19. Anlage nach einem der Ansprüche 14 bis 18, dadurch gekennzeichnet, dass der Eintritt des Gefässes mit einer divergierenden Vorrichtung (106) versehen ist, während vorteilhaft der Austritt mit einer jeweils den Eingang und den Ausgang der Erzeugnisse gegenüber dem Gefäss (2) erleichternden konvergierenden Vorrichtung (108) versehen ist.

FIG. 1

EP 0 192 900 B1

FIG.2

Fig.3

POMPE

14a
14b
18
14
16
100
12
102
10
104
105
106
116
108
118
30
112
107
111
3
8
2
1

Fig. 6

Fig.5